(19) Europäisches Patentamt
European Patent Office
Office européen des brevets

(11) Veröffentlichungsnummer : **0 371 331 B1**

(12) # EUROPÄISCHE PATENTSCHRIFT

(45) Veröffentlichungstag der Patentschrift :
**02.01.92 Patentblatt 92/01**

(51) Int. Cl.$^5$ : **B01J 23/84,** C07C 209/18, C07C 209/36

(21) Anmeldenummer : **89121178.1**

(22) Anmeldetag : **16.11.89**

(54) **Kupfer-Trägerkatalysator, Verfahren zu seiner Herstellung und Verfahren zur Herstellung von N-alkylierten aromatischen Aminen unter Einsatz dieses Kupfer-Katalysators.**

(30) Priorität : **29.11.88 DE 3840194**

(43) Veröffentlichungstag der Anmeldung :
**06.06.90 Patentblatt 90/23**

(45) Bekanntmachung des Hinweises auf die Patenterteilung :
**02.01.92 Patentblatt 92/01**

(84) Benannte Vertragsstaaten :
**DE FR GB**

(56) Entgegenhaltungen :
**EP-A- 0 009 590**
**EP-A- 0 266 807**

(56) Entgegenhaltungen :
**FR-A- 1 296 017**
**GB-A- 1 413 949**
**US-A- 2 580 284**
**US-A- 4 402 869**

(73) Patentinhaber : **BAYER AG**
**W-5090 Leverkusen 1 Bayerwerk (DE)**

(72) Erfinder : **Immel, Otto, Dr.**
**Immenhofweg 26**
**W-4150 Krefeld (DE)**
Erfinder : **Schwarz, Hans-Helmut, Dr.**
**Ratherstrasse 90**
**W-4150 Krefeld (DE)**
Erfinder : **Braden, Rudolf, Dr.**
**Nothauser Feld 1**
**W-5068 Odenthal (DE)**

EP 0 371 331 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen neuen Kupfer-Trägerkatalysator, ein Verfahren zu seiner Herstellung und ein Verfahren zur Herstellung von N-alkylierten aromatischen Aminen unter Einsatz dieses Kupfer-Katalysators. In bevorzugter Weise können die zu alkylierenden Amine simultan im Alkylierungsreaktor aus den zugrundeliegenden Nitroverbindungen unter gleichzeitigem Einsatz von Wasserstoff hergestellt werden.

Es wurde ein neuer Kupfer-Katalysator auf einem Träger aus $\alpha$-$Al_2O_3$ oder $\gamma$-$Al_2O_3$, der mit Verbindungen des Mangans und eines oder mehrerer Seltenerdmetalle imprägniert ist, mit einem Cu-Gehalt von 0,1-5 Gew.-%, bevorzugt 0,1-3 Gew.-%, besonders bevorzugt 1-3 Gew.-%, einem Gesamtgehalt an Verbindungen des Mangans und des (der) Seltenerdmetalles (-metalle), gerechnet als Metalle, von 0,05 bis 8 Gew.-%, bevorzugt 0,2-5 Gew.-%, wobei das Gewichtsverhältnis von Seltenerdmetall(en) zu Mangan, als Metall gerechnet, 5 : 1-1: 5, bevorzugt 10 : 9-1 : 2, beträgt und einem zusätzlichen Gehalt an einem oder mehreren Alkalihydroxid(en) von 1-6 Gew.-%, bevorzugt 2-5 Gew.-%, wobei alle Prozentwerte auf das Gesamtgewicht des Katalysators bezogen sind, gefunden.

Die erfindungsgemäßen Katalysatoren sind demnach dadurch gekennzeichnet, daß sie das Kupfer auf einem in spezieller Weise imprägnierten $Al_2O_3$-Träger enthalten, wodurch es eine spezielle Aktivität entfaltet. Der erfindungsgemäße Katalysator kann demzufolge auf andere katalytisch aktive Metalle verzichten.

Als $Al_2O_3$-Träger kommt $\alpha$-$Al_2O_3$ oder $\gamma$-$Al_2O_3$, bevorzugt $\gamma$-$Al_2O_3$ in Frage.

Ein solcher Träger wird mit Verbindungen des Mangans und eines oder mehrerer Seltenerdmetalle imprägniert, wobei die oben angegebenen Mengen und Gewichtsverhältnisse in Frage kommen. Als Seltenerdmetalle (III. Nebengruppe des Periodensystems der Elemente) seien hierbei genannt :
Scandium, Yttrium, Lanthan und die Lanthaniden. In bevorzugter Weise seien Yttrium, Lanthan, Cer, Praseodym, Neodym und Dysprosium, in besonders bevorzugter Weise Cer und Lanthan und in ganz besonders bevorzugter Weise Cer verstanden. Das Cer kann hierbei mit anderen Lanthaniden, beispielsweise mit Lanthan, Praseodym, Neodym, Dysprosium, oder mit Yttrium vergesellschaftet sein. Eine solche Vergesellschaftung ist dem Fachmann im übrigen für alle genannten seltenen Erdmetalle geläufig.

Der erfindungsgemäße Katalysator ist ferner durch den genannten Gehalt an Kupfer auf dem beschriebenen imprägnierten Träger gekennzeichnet.

Weiterhin weist der Katalysator einen zusätzlichen Gehalt an Alkalimetallhydroxid von 1-6 Gew.-%, bevorzugt 2-5 Gew.-%, bezogen auf das Gesamtgewicht des Katalysators, auf. Alkalimetallhydroxide sind Lithiumhydroxid, Natriumhydroxid, Kaliumhydroxid, Rubidiumhydroxid, Cäsiumhydroxid oder ein Gemisch von ihnen, bevorzugt Lithiumhydroxid, Natriumhydroxid, Kaliumhydroxid oder ein Gemisch von ihnen, besonders bevorzugt Natriumhydroxid, Kaliumhydroxid oder ein Gemisch von ihnen.

Zur Herstellung des Katalysators kann der Träger in pulverförmiger oder stückiger Form eingesetzt werden.

Für den Fall des Einsatzes des erfindungsgemäßen Katalysators im Festbettverfahren ist die stückige Form bevorzugt. Als stückige Form seien beispielsweise Strangpreßlinge, Pillen oder Kugeln mit Abmessungen von etwa 2-10 mm genannt.

Zur Herstellung des erfindungsgemäßen Katalysators wird ein solcher Träger mit Verbindungen des Cers und Mangans beaufschlagt, der so beaufschlagte Träger nach dem Trocknen auf 200-450°C, bevorzugt 250 bis 430°C erhitzt und ist danach vorbereitet für die weitere Behandlung mit einem Kupfersalz, das aufgetränkt oder aufgesprüht werden kann, wonach sich eine erneute Trocknungsphase anschließt. Das Aufbringen des (der) Seltenerdmetalles (-metalle) und des Mangans auf den Katalysatorträger kann beispielsweise durch bloßes Tränken oder Sprühen mit geeigneten Salzen dieser Metalle erfolgen, wonach sich eine Trocknungsphase und die genannte Erhitzungsphase anschließen. Das Trocknen wird in einer dem Fachmann bekannten Weise bei 80-130°C, gegebenenfalls in einem Vakuumtrockenschrank, vorgenommen. Bei der nachfolgenden Erhitzungsphase werden die Metallsalze in fest auf dem Katalysatorträger haftende Verbindungen übergeführt. Das Aufbringen der genannten Metalle kann jedoch auch durch gemeinsames Ausfällen eines Metallhydroxidgemisches aus Salzen der genannten Metalle auf dem Träger mit Hilfe von Alkalilauge oder Ammoniak und gegebenenfalls anschließendem Auswaschen der löslichen Anteile mit Wasser erfolgen. Als Salze der genannten Metalle kommen insbesondere die Sulfate, Chloride, Acetate und/oder Nitrate in Betracht.

Das anschließende Trocknen und Erhitzen in den genannten Temperaturbereichen erfolgt jeweils in einer Zeit von 1-120 Stunden ; während dieser Zeit kann die Temperatur in den angegebenen Bereichen von niederen auf höhere Werte erhöht werden. Nach der beschriebenen Erhitzungsphase (Temperung) wird der mit dem genannten Metallverbindungen imprägnierte Katalysatorträger mit einer Kupfer enthaltenden Lösung getränkt. Hierbei kann so vorgegangen werden, daß das Kupfer, beispielsweise in Form wäßriger Lösungen des Chlorids, Nitrats, Acetats oder eines anderen geeigneten Salzes auf den Träger aufgetränkt oder aufgesprüht wird, gefolgt von einer Trocknung. Man kann jedoch auch vor der Trocknung den mit Kupfer getränkten Träger mit einer Lösung der genannten basischen Verbindungen behandeln, wobei das Kupfer als Oxid oder Hydroxid

ausfällt. Auch hier schließt sich eine Trocknung an, die unter den obengenannten Bedingungen durchgeführt werden kann. Schließlich wird der Katalysator mit einer Lösung eines oder mehrerer Alkalimetallhydroxid(e) beaufschlagt, daß ein Gehalt daran in der beschriebenen Menge vorliegt.

Danach steht ein solcher erfindungsgemäßer Katalysator grundsätzlich zum Einsatz zur Verfügung. In bevorzugter Weise wird er jedoch vor seinem Einsatz, besonders bevorzugt nach Anordnung im für seinen Einsatz vorgesehenen Reaktor, durch Behandlung mit Wasserstoff bei einer Temperatur von 150-350°C aktiviert. Nach der Aktivierung kann es wünschenswert sein, Anionen wie Chlorid, Nitrat, Acetat oder andere und gegebenenfalls die Kationen der zur Ausfällung benutzten basischen Verbindungen durch eine Wasserwäsche zu entfernen, die auch bei vorher vorgenommener Anordnung des Katalysators im Reaktor vorgenommen werden kann.

Man kann jedoch auch den mit den genannten Metallverbindungen imprägnierten Katalysator zunächst mit einer Lösung einer der genannten basischen Verbindungen tränken, anschließend trocknen und auf den so vorbehandelten, basisch eingestellten Katalysatorträger Lösungen von Kupfersalzen auftragen, wobei im Moment der Tränkung auch die Ausfällung des Kupfers in Form seines Oxids oder Hydroxids erfolgt. Auch hierbei ist nach einer anschließenden Trocknung der Katalysator grundsätzlich einsatzbereit, kann jedoch bevorzugt in der beschriebenen Weise vorab mit Wasserstoff bei der genannten Temperatur aktiviert werden.

Ein zur Fällung des Kupfers als Oxid oder Hydroxid mit basischen Verbindungen beaufschlagter Katalysator ist grundsätzlich in Gegenwart der Reste solcher alkalischer Verbindungen betriebsbereit. Der Gehalt solcher alkalischer Verbindungen wurde bereits oben genannt.

Die Tränkung oder das Besprühen des $Al_2O_3$-haltigen Trägers mit den genannten Stoffen und die hierfür erforderlichen Arbeitsgeräte sind dem Fachmann bekannt ; ebenso bekannt ist die Einstellung der gewünschten Beaufschlagung durch die Wahl der Menge und Konzentration der Lösungen der genannten Metallverbindungen.

Als Fixierung des Kupfers auf den Träger sei beispielsweise die Überführung in unlösliche Oxide und/oder Hydroxide des Kupfers oder die Reduktion des Kupfersalzes in elementares Kupfer mit Hilfe von Wasserstoff genannt.

Gegebenenfalls kann ein Teil des Kupfers in Form der Oxide/Hydroxide und ein weiterer Teil in Form von metallischem Kupfer vorliegen.

Der erfindungsgemäße Katalysator eignet sich in hervorragender Weise zur Herstellung von sekundären und/oder tertiären aromatischen Aminen durch N-Alkylierung aromatischer Amine mit Alkanolen.

Es ist bereits bekannt, zur Herstellung von N-alkylierten aromatischen Verbindungen aromatische Amine mit Alkanolen oder den zugehörigen Dialkylethern in der Gasphase umzusetzen. Als Katalysatoren werden beispielsweise Aluminiumoxid oder Silikate vorgeschlagen (beispielsweise in DE-PS 638756 Silikate, wie Tonsil). Die genannten Katalysatoren haben den Nachteil, daß ihre Aktivität bei der Alkylierung von aromatischen Aminen schnell nachläßt (vgl. Houben-Weyl, Methoden der organischen Chemie, Band XI/1 (1957), S. 126).

Weiterhin ist aus DE-PS 617990 und aus DE-OS 2335906 bekannt, daß man für die genannte Aufgabe Trägerstoffe verwenden kann, die Sauerstoffsäuren des Phosphors enthalten. Lebensdauer und Selektivität solcher Katalysatoren sind für technische Verfahren jedoch nicht befriedigend.

Weiterhin wurde in US 2.580.284 vorgeschlagen, Anilin und Methanol unter Verwendung eines Katalysators, der metallisches Kupfer auf $Al_2O_3$ und weitere Oxide, wie Zinkoxid, Cadmiumoxid, Eisenoxid, Chromoxid, Calciumoxid, Magnesiumoxid oder Kaliumoxid enthält, umzusetzen Solche Katalysatoren leiden unter der Abscheidung teerartiger Substanzen bereits nach relativ kurzer Zeit.

Ein weiteres Verfahren zur Umsetzung von Anilin mit Methanol zu N-Methylanilin nach DE-OS 2061709 wird an einem Chrom-Katalysator durchgeführt, der Kupfer, Zink, Eisen, Nickel oder Molybdän sowie Barium, Magnesium oder Mangan enthalten kann. Dieses Verfahren hat den Nachteil, daß es unter einem Druck von 50-150 bar durchgeführt wird und daher für eine industrielle Anwendung zu kostenaufwendig ist ; auch dieser Katalysator erreicht keine ausreichende Lebensdauer.

Gemäß DE-OS 2120641 werden Kupferchromit-Katalysatoren mit Barium, Mangan, Cer und anderen als Promotoren zur Herstellung von sekundären oder tertiären aromatischen Aminen aus aliphatischen Alkoholen und aromatischen Nitroverbindungen eingesetzt. Dieses Verfahren führt aber nur zu unbefriedigenden Ausbeuten.

Bei der Vielzahl der bisher untersuchten Katalysatoren ist es daher überraschend, daß der erfindungsgemäße Katalysator unter Benutzung eines speziell imprägnierten Trägers und bei Verzicht auf Chrom als aktivem Katalysatorbestandteil die genannten Nachteile vermeidet.

Die Erfindung betrifft daher weiterhin ein Verfahren zur Herstellung von aromatischen Aminen der Formel

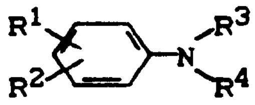

(I),

in der

$R^1$ und $R^2$ unabhängig voneinander Wasserstoff oder geradkettiges oder verzweigtes $C_1$-$C_5$-Alkyl oder $C_1$-$C_5$-Alkoxy bedeuten und gemeinsam auch einen anellierten Benzolring bedeuten können und
$R^3$ und $R^4$ unabhängig voneinander für geradkettiges oder verzweigtes $C_1$-$C_{10}$-Alkyl oder $C_3$-$C_{10}$-Cycloalkyl stehen, wobei $R^4$ zusätzlich für Wasserstoff stehen kann,

durch katalysierte Umsetzung von aromatischen Aminen der Formel

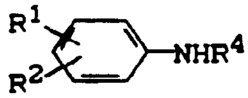

(II)

mit Alkanolen der Formel

$R^3OH$ (III)

oder den zugehörigen Ethern $R^3$-O-$R^3$, worin
$R^3$ und $R^4$ die obige Bedeutung haben, bei erhöhter Temperatur, das dadurch gekennzeichnet ist, daß man bei 160-400°C in der Gasphase in Gegenwart des oben beschriebenen erfindungsgemäßen Katalysators arbeitet, wobei 0,5-20 Mol Alkanol pro Mol des aromatischen Amins eingesetzt werden.

Als geradkettiges oder verzweigtes $C_1$-$C_5$-Alkyl oder $C_1$-$C_5$-Alkoxy seien beispielsweise genannt : Methyl, Ethyl, Propyl, Isopropyl, Butyl, Isobutyl, die verschiedenen Amylreste, Methoxy, Ethoxy, Propoxy, Isopropoxy, Butoxy, Isobutoxy, die verschiedenen Amyloxyreste. In bevorzugter Weise seien als solche Reste Methyl, Ethyl, Methoxy und Ethoxy, besonders bevorzugt Methyl und Methoxy genannt. $C_3$-$C_{10}$-Cycloalkyl ist beispielsweise Cyclopropyl, Cyclobutyl, Cyclopentyl, Cyclohexyl, Cycloheptyl, Cyclooctyl, Methyl-cyclopentyl, Methylcyclohexyl, 4-tert.-Butyl-cyclohexyl, Menthyl, bevorzugt, Cyclopropyl, Cyclopentyl und Cyclohexyl. In ganz besonders bevorzugter Weise wird von im Kern nicht substituierten aromatischen Aminen ausgegangen.

In weiterhin bevorzugter Weise wird von aromatischen Aminen der Formel

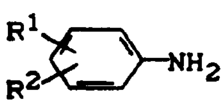

(IV)

ausgegangen, worin
$R^1$ und $R^2$ die obige Bedeutung haben.

In weiterhin bevorzugter Form wird von aromatischen Aminen der Formel

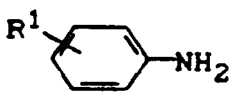

(V)

ausgegangen, worin
$R^1$ die obige Bedeutung hat.

Eine beispielhafte, keineswegs erschöpfende Aufzählung von aromatischen Aminen für das erfindungsgemäße Verfahren ist die folgende : Anilin, 1-Naphthylamin, o-, m-, p-Toluidin, o-, m-, p-Ethylanilin, die Isomeren Xylidine, wie 2,3-, 2,4-, 2,5-, 2,6-, 3,4- oder 3,5-Dimethylanilin, 4-Methyl-naphthylamin-1.

Das erfindungsgemäße Verfahren wird in der Gasphase bei einer Temperatur von 160-400°C, bevorzugt 160-350°C, einem Druck von 0,5-10 bar, bevorzugt 0,5 bis 3 bar, und einer Katalysatorbelastung von 0,1-2 kg Ausgangsmaterial je Liter Katalysator und Stunde durchgeführt. In bekannter Weise stehen die Katalysatorbelastung und die sich daraus ergebende Verweilzeit in reziprokem Verhältnis zueinander.

Das als Ausgangsmaterial eingesetzte aromatische Amin wird vor Eintritt in den Reaktionsraum verdampft und über den als Festbett angeordneten Katalysator geleitet. In bevorzugter Weise wird das verdampfte Ausgangsmaterial mit einem Trägergasstrom verdünnt beispielsweise mit $H_2$, $N_2$, $H_2O$-Dampf, $CH_4$ und anderen inerten Gasen.

Alkanole für das erfindungsgemäße Verfahren sind offenkettige Alkanole mit 1-10 C-Atomen, bevorzugt mit 1-6 C-Atomen, besonders bevorzugt mit 1-2°C-Atomen und cyclische Alkanole mit 3-10 C-Atomen bevorzugt mit 3, 5 oder 6 C-Atomen, wie Methanol, Ethanol, Propanol, i-Propanol, Butanol, i-Butanol, tert.-Butanol, Pentanol, i-Pentanol, die isomeren Hexanole, Heptanole, Octanole oder Decanole, Cyclopropanol, Cyclobutanol, Cyclopentanol, Cyclohexanol, Cycloheptanol oder Cyclooctanol, sowie 2-, 3- oder 4-Methyl-cyclohexanol, 2-Ethyl-cyclohexanol, 3,3,5-Trimethylcyclohexanol, 4-tert.-Butylcyclohexanol und Menthol ($C_{10}$).

Anstelle der Alkohole können auch die zugängigen Ether $R^3$-O-$R^3$ eingesetzt werden ; der Einsatz der Alkanole ist jedoch bevorzugt.

Das Molverhältnis des Alkanols zu dem zu N-alkylierenden aromatischen Amin beträgt 0,5-20 :1, bevorzugt 0,7-15 :1. Beim Einsatz der zugehörigen Ether ist für die Berechnung des Molverhältnisses 1 Mol $R^3$-O-$R^3$ 2 Molen $R^3OH$ gleichzusetzen. Mit solchen molaren Mengen an Alkanol können aus sekundären aromatischen Aminen der Formel (II), in denen also $R^4$ verschieden von Wasserstoff ist, tertiäre aromatische Amine der Formel (I) hergestellt werden, in denen $R^4$ verschieden von Wasserstoff ist. Weiterhin können aus primären aromatischen Aminen der Formel (II), in denen $R^4$ für Wasserstoff steht oder aus aromatischen Aminen der Formeln (IV) bzw. (V) sekundäre oder tertiäre Amine oder ein Gemisch von ihnen hergestellt werden, in denen also das Amin-N-Atom einfach oder zweifach alkyliert wird. Für den Fall der nur einfachen N-Alkylierung ist es weiterhin bevorzugt, einen noch engeren molaren Bereich von 0,8-5 Mol Alkanol pro Mol primäres aromatisches Amin einzusetzen. Da die Trennung von am N-Atom einfach und zweifach alkylierten aromatischen Amine häufig schwierig ist, ist man bei der Herstellung nur monoalkylierter aromatischer Amine bestrebt, den Anteil an 2-fach am N-Atom alkylierten Aminen soweit wie möglich zu unterdrücken. Diesem Bestreben kommt in überraschend guter Weise der Einsatz des oben beschriebenen erfindungsgemäßen Katalysators entgegen. Hierbei wurde überraschend gefunden, daß auch noch bei relativ hohen molaren Mengen an Alkanol, nämlich bis zu 5 Mol pro Mol des primären aromatischen Amins fast ausschließlich am N-Atom monoalkylierte aromatische Amine entstehen, wenn man im unteren Temperaturbereich und im unteren Bereich der Verweilzeiten bleibt. Hierfür seien beispielsweise genannt : 160-220°C und Verweilzeiten von 0,5 bis 8 sec.

Umgekehrt führen höhere Reaktionstemperaturen und längere Verweilzeiten, beispielsweise 230-300°C und 5 bis 20 sec. in Verbindung mit höheren molaren Überschüssen an Alkanol im genannten Bereich zu einer fast ausschließlichen Dialkylierung am N-Atom.

In einer bevorzugten Variante des erfindungsgemäßen Verfahrens wird nicht von aromatischen Aminen der Formel (IV) sondern von den zugrundeliegenden aromatischen Nitroverbindungen der Formel

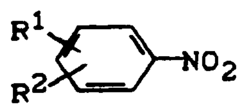

(VI)

ausgegangen, worin

$R^1$ und $R^2$ den obigen Bedeutungsumfang haben.

In diesem Falle ist mindestens die zur Reduktion der Nitrogruppe zur Aminogruppe erforderliche Menge Wasserstoff zusätzlich einzusetzen, der auch in diesem Falle gleichzeitig als Trägergasstrom dient. In bevorzugter Weise wird jedoch überschüssiger Wasserstoff eingesetzt, der nach Abtrennung vom Reaktionsprodukt recyclisiert wird. Als ein solcher Überschuß sei beispielsweise 150 bis 1000% der stöchiometrisch zur Reduktion der Nitrogruppe erforderlichen Wasserstoffmenge genannt. Dem Wasserstoff können zur Verdünnung die obengenannten Inertgase zugesetzt werden.

Eine beispielhafte, keineswegs erschöpfende Aufzählung von geeigneten aromatischen Nitroverbindungen für das erfindungsgemäße Verfahren ist die folgende : Nitrobenzol, o-, m-, p-Methyl-nitrobenzol, o-, m-, p-Ethyl-nitrobenzol.

N-alkylierte aromatische Amine, die erfindungsgemäß hergestellt werden können, sind Ausgangsstoffe zur Erzeugung von Farbstoffen, Schädlingsbekämpfungsmitteln oder anderen biologischen Wirkstoffen, wie Pflanzenschutzmitteln, Wachstumsregulatoren usw. Ferner dienen sie als Mineralölzusätze oder als Zusätze für Lacke oder andere polymere Systeme.

Beispiel 1

200 g eines handelsüblichen $\gamma$-$Al_2O_3$ mit einer spezifischen Oberfläche von 350 $m^2/g$ und einem Kugeldurchmesser von 2 bis 6 mm wurden mit einer Lösung getränkt, die aus 12,4 g $Ce(NO_3)_3 \cdot 6\ H_2O$, 18,28 g $Mn(NO_3)_2 \cdot 4\ H_2O$ und 50 g Wasser hergestellt worden war. Das getränkte $Al_2O_3$ wurde im Wasserstrahlvakuum 18 Stunden bei 120°C getrocknet und anschließend 3 Stunden lang bei 400°C getempert. Der so hergestellte Katalysatorträger wurde mit 70 g einer wäßrigen $Cu(NO_3)_2$-Lösung getränkt, die 4 g Cu enthält. Der feuchte Katalysator wurde getrocknet und 3 Stunden im Wasserstoffstrom (100 l $H_2O$/h) bei 350°C aktiviert. Anschließend wurde 58,8 g des Katalysators mit einer Lösung von 1,2 g NaOH in 20 g Wasser nochmals getränkt und wieder getrocknet. Weitere 58,8 g des aktivierten Katalysators wurden mit einer Lösung von 1,2 g KOH in 20 g Wasser getränkt und ebenfalls wieder getrocknet.

Beispiel 2

20 ml (17,4 g) des nach Beispiel 1 hergestellten Katalysators, der mit KOH nachbehandelt worden war, wurden in ein Reaktionsrohr eingefüllt, das einen Innendurchmesser von 17 mm und eine Länge von 60 mm hatte. Das Reaktionsrohr wude durch eine elektrische Beheizung auf 250°C gehalten, während unter Benutzung einer geeichten Dosiervorrichtung innerhalb von 15,5 Stunden 258 g eines Gemisches aus Ethanol und Nitrobenzol und 20 l $H_2$/h in das Reaktionsrohr geleitet wurde. In dem Einsatzgemisch lagen Ethanol und Nitrobenzol im Molverhältnis 4 : 1 vor. Das Reaktionsprodukt wurde kondensiert und gaschromatographisch analysiert. Der Wasseranteil wurde dabei nicht erfaßt ; der organische Anteil des Reaktionsproduktes ergab folgende Zusammensetzung :

| | |
|---|---|
| Nitrobenzol: | 0,3 % |
| Anilin: | 5,7 % |
| N-Methylanilin: | 90,6 % |
| N,N-Dimethylanilin: | 3,2 % |
| Nebenprodukte: | 0,2 % |

Beispiel 3

Unter Benutzung der gleichen Apparatur und der gleichen Katalysatormenge wie in Beispiel 2 wurden m-Nitrotoluol und Ethanol im Molverhältnis 1 : 4 und 20 l $H_2$/h eingesetzt. Es wurde eine Reaktionstemperatur von 220°C eingestellt. Die Katalysatorbelastung betrug 0,81 g Mischung/ml Kat. × h. Das im Verlauf von 16 Stunden entstandene Reaktionsprodukt hatte laut Gaschromatographie folgende Zusammensetzung :

| | |
|---|---|
| m-Toluidin : | 0,7 % |
| N-Ethyl-m-toluidin: | 90,6 % |
| N,N-Diethyl-m-toluidin: | 6,1 % |
| Nebenprodukte: | 2,6 % |

Beispiel 4

Unter Benutzung der gleichen Apparatur und des gleichen Katalysators wie in den Beispielen 2 und 3 wurden Anilin und Ethanol im Molverhältnis 1 : 4 eingesetzt. Als Trägergas diente Wasserstoff (20 l/h). Bei einer Reaktionstemperatur von 200°C und einer Katalysatorbelastung von 0,72 g Ethanol-Anilin-Gemisch/ml Kat. × h entstand im Verlauf von 63 Stunden ein Reaktionsgemisch folgender Zusammensetzung :

| | |
|---|---|
| Anilin: | 3,1 % |
| N-Ethananilin: | 95,6 % |
| N,N-Diethylanilin: | 0,5 % |
| Nebenprodukte: | 0,8 % |

Beispiel 5

30 ml (25,6 g) des nach Beispiel 1 angefertigten Cu-Ce-Mn/$Al_2O_3$/NaOH-Katalysators wurden zur Umsetzung von Nitrobenzol mit Methanol bei 250°C (wie in Beispiel 2) benutzt. Eingesetzt wurde eine Mischung, die Nitrobenzol und Methanol im Molverhältnis 1 : 4 enthielt. Es wurden 20 l $H_2$ pro Stunde über den Katalysator geleitet. Die eingesetzte Methanol-Nitrobenzol-Mischung wurde mit einer Katalysatorbelastung von 0,42 g/ml.h zugefügt. Die kontinuierlich durchgeführte Reaktion erbrachte in 74 Stunden ein Reaktionsgemisch folgender Zusammensetzung :

| | |
|---|---|
| Anilin: | 25,7 % |
| N-Methylanilin | 72,5 % |
| N,N-Dimethylanilin: | 1,6 % |
| Nebenprodukte: | 0,2 % |

**Patentansprüche**

1. Kupfer-Katalysator auf einem Träger aus $\alpha$-$Al_2O_3$ oder $\gamma$-$Al_2O_3$, der mit Verbindungen des Mangans und eines oder mehrerer Seltenerdmetalle imprägnert ist, mit einem Cu-Gehalt von 0,1-5 Gew.-%, einem Gesamtgehalt an Verbindungen des Mangans und des (der) Seltenerdmetalles (-metalle), gerechnet als Metalle, von 0,05-8 Gew.-%, wobei das Gewichtsverhältnis von Seltenerdmetall(en) zu Mangan, als Metall gerechnet, 5 : 1-1 : 5, beträgt und einem zusätzlichen Gehalt an einem oder mehreren Alkalihydroxid(en) von 1-6 Gew.-%, wobei alle Prozentwerte auf das Gesamtgewicht des Katalysators bezogen sind.

2. Katalysator nach Anspruch 1, gekennzeichnet durch einen Cu-Gehalt von 0,1-3 Gew.-%.

3. Katalysator nach Anspruch 1, gekennzeichnet durch einen Gehalt an Lithiumhydroxid, Natriumhydroxid, Kaliumhydroxid oder einem Gemisch von ihnen,

4. Katalysator nach Anspruch 1, herstellbar durch Behandeln des Trägers mit löslichen Verbindungen des Mangans und des (der) Seltenerdmetalles (-metalle), Überführung dieser Verbindungen in auf dem Träger fest haftende Verbindungen durch Trocknen und anschließendes Tempern bei 200-450°C, bevorzugt bei 250-430°C, Tränkung des so erhaltenen getemperten Trägers mit einem löslichen Kupfersalz, Fixierung des Kupfers auf dem Träger, Beaufschlagung mit einem oder mehreren Alkalimetallhydroxid(en) und gegebenenfalls Aktivierung durch Behandlung mit Wasserstoff bei 150-350°C.

5. Katalysator nach Anspruch 4, gekennzeichnet durch das Auftränken von 1-6 Gew.-%, bezogen auf das Gesamtgewicht des Katalysators, eines oder mehrerer Alkalimetallhydroxide nach der Aktivierung.

6. Katalysator nach Anspruch 4, bei dem die aktivierende Behandlung mit Wasserstoff erst unmittelbar vor seinem Einsatz in dem hierfür vorgesehenen Reaktor durchgeführt wird.

7. Verfahren zur Herstellung von aromatischen Aminen der Formel

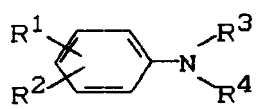 ,

in der

R[1] und R[2] unabhängig voneinander Wasserstoff oder geradkettiges oder verzweigtes $C_1$-$C_5$-Alkyl oder $C_1$-$C_5$-Alkoxy bedeuten und gemeinsam auch einen anellierten Benzolring bedeuten können und

$R^3$ und $R^4$ unabhängig voneinander für geradkettiges oder verzweigtes $C_1$-$C_{10}$-Alkyl oder $C_3$-$C_{10}$-Cycloalkyl stehen, wobei $R^4$ zusätzlich für Wasserstoff stehen kann,

durch katalysierte Umsetzung von aromatischen Aminen der Formel

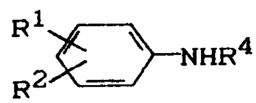

mit Alkanolen der Formel

$R^3OH$

oder den zugehörigen Ethern $R^3$-O-$R^3$, worin
$R^3$ und $R^4$ die obige Bedeutung haben, bei erhöhter Temperatur, dadurch gekennzeichnet, daß man bei 160-400°C in der Gasphase in Gegenwart eines Kupfer-Katalysators auf einem Träger aus $\alpha$-$Al_2O_3$ oder $\gamma$-$Al_2O_3$, der mit Verbindungen des Mangans und eines oder mehrerer Seltenerdmetalle imprägniert ist, mit einem Cu-Gehalt von 0,1-5 Gew.-%, einem Gesamtgehalt an Verbindungen des Mangans und des (der) Seltenerdmetalles (-metalle), gerechnet als Metalle, von 0,05-8 Gew.-%, wobei das Gewichtsverhältnis von Seltenerdmetall(en) zu Mangan als Metall gerechnet, 5 : 1-1 : 5 beträgt und einem zusätzlichen Gehalt an einem oder mehreren Alkalihydroxid(en) von 1-6 Gew.-%, wobei alle Prozentwerte auf das Gesamtgewicht des Katalysators bezogen sind, arbeitet, wobei 0,5-20 Mol Alkanol pro Mol des aromatischen Amins eingesetzt werden.

8. Verfahren nach Anspruch 7, dadurch gekennzeichet, daß aromatische Amine der Formel

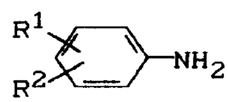

eingesetzt werden, in der
$R^1$ und $R^2$ unabhängig voneinander Wasserstoff, geradkettiges oder verzweigtes $C_1$-$C_5$-Alkyl oder $C_1$-$C_5$-Alkoxy bedeuten.

9. Verfahren nach Anspruch 8, dadurch gekennzeichnet, daß das aromatische Amin im Alkylierungsreaktor bei gleichzeitig zugesetztem Wasserstoff aus der zugrundeliegenden Nitroverbindung der Formel

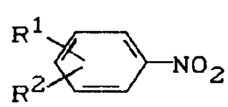

hergestellt wird.

10. Verfahren nach Anspruch 7, dadurch gekennzeichnet, daß die Gasphasenreaktion bei einem Druck von 0,5 bis 10 bar durchgeführt wird.

## Claims

1. Copper catalyst on a carrier of $\alpha$-$Al_2O_3$ or $\gamma$-$Al_2O_3$ which is impregnated with compounds of manganese and of one or more rare earth metals and has a Cu content of 0.1-5% by weight, a total content of compounds of manganese and of the rare earth metal or metals of 0.05-8% by weight, calculated as metals, the weight ratio of rare earth metal(s) to manganese being 5 : 1-1 : 5, calculated as metal, and an additional content of 1-6% by weight of one or more alkali metal hydroxides, all percentages being relative to the total weight of the catalyst.

2. Catalyst according to Claim 1, characterized by a Cu content of 0.1-3% by weight.

3. Catalyst according to Claim 1, characterized by a content of lithium hydroxide, sodium hydroxide, potassium hydroxide or a mixture of these.

4. Catalyst according to Claim 1, which can be prepared by treatment of the carrier with soluble compounds of manganese and of the rare earth metal or metals, conversion of these compounds into compounds which adhere firmly to the carrier, by drying and subsequent heating at 200-450°C, preferably at 250-430°C, impregnation of the heated carrier thus obtained with a soluble copper salt, fixing of the copper on the carrier, treating with one or more alkali metal hydroxides and, if appropriate, activation by treatment with hydrogen at 150-350°C.

5. Catalyst according to Claim 4, characterized by application by impregnation of 1-6% by weight, relative to the total weight of the catalyst, of one or more alkali metal hydroxides after the activation.

6. Catalyst according to Claim 4, in which the activating treatment with hydrogen is not carried out until directly before the catalyst is used in the reactor intended for this purpose.

7. Process for the preparation of aromatic amines of the formula

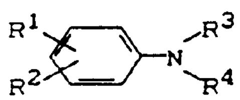 ,

in which

$R^1$ and $R^2$ independently of one another denote hydrogen or straight-chain or branched $C_1$-$C_5$-alkyl or $C_1$-$C_5$-alkoxy and together may also denote a fused benzene ring, and

$R^3$ and $R^4$ independently of one another represent straight-chain or branched $C_1$-$C_{10}$-alkyl or $C_3$-$C_{10}$-cycloalkyl, and $R^4$ can additionally represent hydrogen,

by catalytic reaction of aromatic amines of the formula

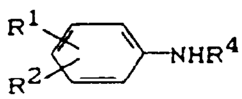

with alkanols of the formula

$R^3OH$

or the associated ethers $R^3$-O-$R^3$, wherein
$R^3$ and $R^4$ have the above meaning, at elevated temperature, characterized in that the reaction is carried out at 160-400°C in the gas phase in the presence of a copper catalyst on a carrier of $\alpha$-$Al_2O_3$ or $\gamma$-$Al_2O_3$ which is impregnated with compounds of manganese and of one or more rare earth metals and has a Cu content of 0.1-5% by weight, a total content of compounds of manganese and of the rare earth metal or metals of 0.05-8% by weight, calculated as metals, the weight ratio of rare earth metal(s) to manganese being 5 : 1-1 : 5, calculated as metal, and an additional content of 1-6% by weight of one or more alkali metal hydroxides, all percentages being relative to the total weight of the catalyst, and 0.5-20 mol of alkanol per mole of the aromatic amine being employed.

8. Process according to Claim 7, characterized in that aromatic amines of the formula

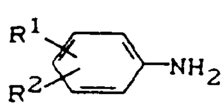

in which
$R^1$ and $R^2$ independently of one another denote hydrogen, straight-chain or branched $C_1$-$C_5$-alkyl or $C_1$-$C_5$-alkoxy, are used.

9. Process according to Claim 8, characterized in that the aromatic amine is prepared in an alkylation reactor with simultaneous addition of hydrogen from the parent nitro compound of the formula

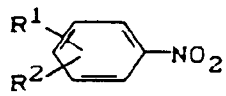

10. Process according to Claim 7, characterized in that the gas-phase reaction is carried out under a pressure of 0.5 to 10 bar.

## Revendications

1. Catalyseur au cuivre fixé sur un support d'α-alumine et ou de γ-alumine, qui est imprégné de composés du manganèse et d'un ou plusieurs des métaux des terres rares, avec une teneur en cuivre de 0,1 à 5% en poids, une teneur totale en composés du manganèse et de métal ou de métaux des terres rares, exprimée en métaux, de 0,05 à 8% en poids, le rapport en poids du métal ou des métaux des terres rares au manganèse, exprimé en métal, s'élevant à 5 : 1-1 : 5, et avec une teneur additionnelle en un ou plusieurs hydroxydes alcalins de 1 à 6% en poids, tous les pourcentages étant rapportés au poids total de catalyseur.

2. Catalyseur suivant la revendication 1, caractérisé par une teneur en cuivre de 0,1 à 3% en poids.

3. Catalyseur suivant la revendication 1, caractérisé par une teneur en hydroxyde de lithium, en hydroxyde de sodium, en hydroxyde de potassium ou en un mélange de ces hydroxydes.

4. Catalyseur suivant la revendication 1, pouvant être préparé par traitement du support avec des composés solubles du manganèse et du ou des métaux des terres rares, transformation de ces composés en composés adhérant fixement au support par séchage puis traitement thermique à 200-450°C, de préférence à 250-430°C, imprégnation du support traité thermiquement de la sorte avec un sel de cuivre soluble, fixation du cuivre sur le support, apport d'un ou plusieurs hydroxydes de métaux alcalins et, le cas échéant, activation par traitement par l'hydrogène à 150-350°C.

5. Catalyseur suivant la revendication 4, caractérisé par l'imprégnation de 1 à 6% en poids, par rapport au poids total de catalyseur, d'un ou plusieurs hydroxydes de métaux alcalins après l'activation.

6. Catalyseur suivant la revendication 4, dans lequel le traitement d'activation par l'hydrogène n'est effectué que juste avant son introduction dans le réacteur prévu à cet effet.

7. Procédé de production d'amines aromatiques de formule

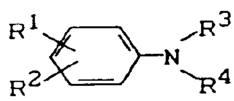 ,

dans laquelle

| | |
|---|---|
| $R^1$ et $R^2$ | représentent, indépendamment l'un de l'autre, l'hydrogène ou un groupe alkyle en $C_1$ à $C_5$ ou un groupe alkoxy en $C_1$ à $C_5$ à chaîne droite ou ramifiée et peuvent aussi représenter ensemble un noyau benzénique condensé et |
| $R^3$ et $R^4$ | représentent indépendamment l'un de l'autre un groupe alkyle en $C_1$ à $C_{10}$ à chaîne droite ou ramifiée ou un groupe cycloalkyle en $C_3$ à $C_{10}$, $R^4$ pouvant représenter en outre l'hydrogène, |

par réaction catalysée d'amines aromatiques de formule

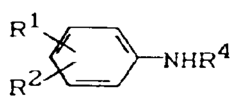

avec des alcanols de formule

$$R^3OH$$

ou avec les éthers $R^3$-O-$R^3$ correspondants, où $R^3$ et $R^4$ ont la définition indiquée ci-dessus,
à température élevée, caractérisé en ce qu'on opère à 160-400°C en phase gazeuse en présence d'un catalyseur au cuivre fixé sur un support d'α-alumine ou de γ-alumine, qui est imprégné de composés du manganèse ou d'un ou plusieurs métaux des terres rares, avec une teneur en cuivre de 0,1 à 5% en poids, une teneur totale en composés du manganèse et du ou des métaux des terres rares, exprimée en métaux, de 0,05 à 8% en poids, le rapport en poids du ou des métaux des terres rares au manganèse, exprimé en métal, s'élevant à 5 : 1-1 : 5 et avec une teneur additionnelle en un ou plusieurs hydroxydes alcalins de 1 à 6% en poids, tous les pourcentages en poids se rapportant au poids total du catalyseur, en utilisant 0,5 à 20 moles d'alcanol par mole de l'amine aromatique.

8. Procédé suivant la revendication 7, caractérisé en ce qu'on utilise des amines aromatiques de formule

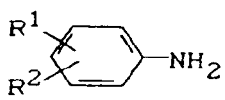

dans laquelle
$R^1$ et $R^2$ représentent indépendamment l'un de l'autre l'hydrogène, un groupe alkyle en $C_1$ à $C_5$ ou un groupe alkoxy en $C_1$ à $C_5$ à chaîne droite ou ramifiée.

9. Procédé suivant la revendication 8, caractérisé en ce que l'amine aromatique est produite dans un réacteur d'alkylation, avec addition simultanée d'hydrogène, à partir du composé nitré de base de formule

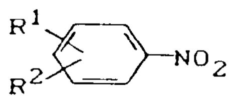

10. Procédé suivant la revendication 7, caractérisé en ce que la réaction en phase gazeuse est conduite à une pression de 0,5 à 10 bars.